# EUROPEAN PATENT APPLICATION

(11) **EP 1 707 199 A1**
(43) Date of publication of application: **04.10.2006**
(21) Application number: 06112006.9
(22) Date of filing: 30.03.2006
(51) Int. Cl.: A61K 31/421, A61K 31/426, A61P 17/00, A61P 17/02, A61P 17/04, A61P 17/06, A61P 17/08

(54) **Oxaprozin or closely related compound for the treatment and prevention of pruritus**

(30) Priority: 30.03.2005 DK 200500437; 30.03.2005 DK 200500438; 27.06.2005 DK 200500948; 27.06.2005 DK 200500949; 27.06.2005 US 694774 P; 28.06.2005 US 695040 P
(71) Applicant: Astion Development A/S, 2100 Copenhagen O (DK)
(72) Inventor: Weidner, Morten Sloth, 2830, Virum (DK)
(74) Representative: Plougmann & Vingtoft A/S

(57) **Abstract**

The invention provides methods and medicaments for the treatment of pruritus in general or pruritus caused by or associated with dermatological diseases including the treatment of the underlying disease by topically administering to skin or by systemically administering to a subject Oxaprozin or a closely related compound or a salt thereof.

## Description

### FIELD OF THE INVENTION

The present invention relates to a pharmacological invention. The invention provides methods and medicaments for the treatment of pruritus in general or pruritus caused by or associated with various events, such as a dermatological disease. The treatment or prevention of pruritus in an individual comprising systemic or topical administration of Oxaprozin or a closely related compound or a salt thereof to said individual.

### BACKGROUND OF THE INVENTION

Pruritus (itching) is a predominant symptom associated with a plethora of skin diseases, but may also be due to systemic causes, such as obstructive jaundice, chronic renal disease, endocrine disease, certain malignancies, and of drug hypersensitivity reactions. Pruritus can be defined subjectively as a localised, non-adapting, usually unpleasant sensation in the skin, which elicits a physiological response resulting in the desire to scratch. The sensation of itch varies significantly, ranging from burning, through pricking, to sensations of insects crawling over the skin. Persistent pruritus may compromise the skin's effectiveness as a protective barrier and cause a serious impairment of quality of life. Furthermore, scratching behaviour is socially disabling.

It is believed that itching sensations may result from the activation of free nerve endings localised at the dermal-epidermal junction by different inflammatory mediators, such as stimulation resulting from the localized release of histamine as well as other mediators, such as vasoactive peptides, enkephalins, substance P, and prostaglandins. Furthermore, the CNS is also thought to play a role in the perception of itch. Therefore, both peripheral and central mechanisms appear to play a role in the relief of pruritus.

Thus, a single pharmacological mechanism cannot explain all causes of pruritus. Histamine released by mast cells may cause pruritus in persons suffering from urticaria and other allergic reactions. Serotonin appears to be a key component of the pruritus that occurs with several diseases, including polycythemia vera, uremia, cholestasis and lymphoma, and of morphine-associated pruritus. Serotonin inhibitors such as cyproheptadine (Periactin), pizotifen, paroxetine (Paxil), and ondansetron (Zofran) have proved effective in treating several of these pruritic conditions. Opioids trigger pruritus in patients receiving intraspinal injections of narcotics. Intravenous and intradermal opioid injections also may induce itching. Narcotic antagonists have been used successfully to relieve pruritus in patients with cholestasis. Atopic dermatitis appears to involve an immune-mediated release of cytokines and other pro-inflammatory agents.

Pruritus has recently been reclassified by Twycross et al. (Quart. J. Med. 2003; 96:7-26) to encompass four different classes termed *pruritoceptive* (due to dermatological conditions like exposure to scabies and funghi), *neuropathic* (due to lesions of afferent pathways of the nervous system, e.g. peripheral neuritis, brain tumours, multiple sclerosis), *neurogenic* (due to centrally acting mediators such as administration of opioids) and *psychogenic.*

Currently, effective anti-pruritus drugs are not available because even the use of strong steroids does not effectively relieve pruritus associated with a number of skin diseases such as atopic dermatitis and contact dermatitis. The only available effective treatment for pruritus is antihistamines, which are only relevant to histamine derived allergic pruritus and in addition has sedative side effects. Most patients suffering from pruritus have another etiology and do not respond to antihistamines. Since there is no effective treatment for most pruritus sufferers, there is a large unmet need in this indication.

It has now been discovered that Oxaprozin, which hitherto has been recognised as a nonsteroidal anti-inflammatory drug (NSAID), effectively inhibits the enzymes protein tyrosine kinase Syk, protein tyrosine kinase ZAP-70 and phosphodiesterase IV (PDE-IV) in pharmacologically relevant doses and effectively reduces the symptoms and inflammation in an animal model of contact dermatitis.

It is generally acknowledged that the term "nonsteroidal anti-inflammatory drugs" is used to describe compounds with a molecular formula based on a substituted phenol or benzene ring and which pharmacologic actions principally are related to the inhibition of the enzyme cyclooxygenase-1 (Cox-1) and to some extent also to the inhibition of cyclooxygenase-2 (Cox-2) *(see* Greaves MW. Pharmacology and significance of nonsteroidal anti-inflammatory drugs in the treatment of skin diseases. J Am Acad Dermatol 1987 April;16(4):751-64*).* Whereas Cox-1 derived prostaglandins are not produced in skin, the Cox-2 enzyme produces prostaglandins at sites of inflammation for which reason the goal of pharmacologic anti-inflammatory therapy in the past has been to inhibit Cox-2 derived prostaglandins.

However, as discovered by the present inventor, Oxaprozin has a quite different pharmaco-dynamic and safe profile in comparison to other known NSAIDs.

The enzymatic activities of each of the enzymes protein tyrosine kinase Syk, protein tyrosine kinase ZAP-70 and phosphodiesterase IV (PDE-IV) are crucial steps in the inflammatory process and operate at a much higher hierarchic levels than the cyclooxygenase pathway. Therefore, Oxaprozin interferes with multiple crucial pathways in the inflammatory cascade, which renders this drug much more promising as an anti-inflammatory candidate, in particular with respect to the treatment of inflammatory dermatological diseases, such as eczemas, than previously expected.

Protein tyrosine kinases Syk and ZAP-70 are a class of enzymes that catalyze the transfer of a phosphate group from ATP to a tyrosine residue located on a protein substrate. They mediate the earliest detectable signalling response of the inflammation process upon activation of mast cells, T cells and B cells leading to multiple cascades of pro-inflammatory reactions. Protein tyrosine kinase Syk is involved in the cellular responses to degranulation, lipid mediator synthesis and cytokine production in inflammatory cells and it is expected that protein tyrosine kinase Syk takes part in allergic or inflammatory reactions through controlling the functions of mast cell, basophils, and eosinophils. The protein tyrosine kinase ZAP-70 is required for T-cell development and T-cell antigen receptor function.

The PDE-IV enzymes belong to the family of phosphodiesterases (PDE's) which are responsible for the hydrolysis of intracellular cyclic adenosine and guanosine monophosphate (cAMP and cGMP, respectively). The type IV phosphodiesterase is a cAMP-specific enzyme localized in airway smooth muscle cells as well as in immune and inflammatory cells. The type IV enzyme is a key enzyme in the hydrolysis of cAMP in mast cells, basophils, eosinophils, monocytes and lymphocytes.

Furthermore, in addition to the enzyme systems recognised by the present inventor, Oxaprozin inhibits both anandamide hydrolase (a fatty acid amide hydrolase) in neurons and NF-kappaB activation in inflammatory cells. Oxaprozin also induces apoptosis of activated monocytes in a dose-dependent manner *(*Dallegri, Franco. A review of the emerging profile of the anti-inflammatory drug oxaprozin. (Expert Opin Pharmacother 2005 May; 6(5):777-85*).*

Obviously, the present inventor has recognised the very great pharmacological potential of Oxaprozin, at least with respect to treating dermatological diseases, in that this single compound is able to modify several of the crucial and principal pathways of the immunological response *in vivo,* which usually requires the administration of several compounds to obtain the same effect.

While Oxaprozin and NSAIDs have been used for a long time in the treatment of systemic inflammatory diseases, like osteoarthritis and rheumatoid arthritis, the utility of Oxaprozin in the treatment of inflammatory dermatological diseases, such as eczemas, have not been emphasized or demonstrated before.

Some NSAIDs have been suggested and developed for the treatment of specific dermatological diseases. To be mentioned is acetylsalicylic acid, indomethacin and parfenac (marketed as bufexamac®) that have been used in the treatment of dermatological diseases for a long time. Acetylsalicylic acid has been used in the treatment of pruritus in atopic eczema; indomethacin has been used in the treatment of sunburned skin; and parfenac has been used in the treatment of eczema, dermatitis and perianal pruritus *(*Greaves MW. Pharmacology and significance of nonsteroidal anti-inflammatory drugs in the treatment of skin diseases, J Am Acad Dermatol 1987 April;16(4):751-64*).*

Salicylic acid has been used in the treatment of psoriasis *(*Going SM, Guyer BM, Jarvie DR, Hunter JA. Salicylic acid gel for scalp psoriasis. Clin Exp Dermatol 1986 May;11 (3):260-2*)* and in the treatment of pruritus *(*Thomsen JS, Simonsen L, Benfeldt E, Jensen SB, Serup J. The effect of topically applied salicylic compounds on serotonin-induced scratching behaviour in hairless rats. Exp Dermatol 2002 August;11(4):370-5).

Topically applied indomethacin has been shown to respond in an animal model of contact dermatitis *(*Lowe NJ, Virgadamo F, Stoughton RB. Anti-inflammatory properties of a prostaglandin antagonist, a corticosteroid and indomethacin in experimental contact dermatitis. BrJ Dermatol 1977 April;96(4):433-8*)* and topical treatment with flubiprofen reduces human skin inflammation *(*Black AK, Hensby CN, Greaves MW. The effect of topical flurbiprofen on human skin inflammation [proceedings]. Br J Clin Pharmacol 1980 January; 9(1):125P*).*

However, some NSAIDS have failed to show any clear ability to treat the inflammation of certain skin diseases. For example, one study demonstrates that topically applied indomethacin has poor effect in relieving the erythema and oedema in moderate to severe inflammation following treatment with cryotherapy. In comparison, the topical application of the steroid, clobetasol propionate, proved to have effect *(*Humphreys F, Spiro J. The effects of topical indomethacin and clobetasol propionate on post-cryotherapy inflammation. BrJ Dermatol 1995 May;132(5):762-5*).* Green and Shuster demonstrate that topical 1% indomethacin had no effect on chronic stable plaque psoriasis in human studies *(*Green CA, Shuster S. Lack of effect of topical indomethacin on psoriasis. BrJ Clin Pharmacol 1987 September;24(3):381-4*).*

Unfortunately, the topical use of various NSAIDs is associated with significant cutaneous side effects. For example, Bufexamac is marketed for the treatment of pruritus and contact allergy, but the compound itself is reported to cause contact allergy. Furthermore, it is reported that aspirin and indometacin may induce urticarial reactions, whereas piroxicam can lead to phototoxic or photoallergic dermatitis. Ketoprofen and Bufexamac are recognised as major contact allergens *(*Gebhardt M and Wollina U. Cutaneous side-effects of nonsteroidal anti-inflammatory drugs (NSAID) in Z Rheumatol 1995 November;54(6):405-12*).* Diclofenac is another NSAID that is associated with cutaneous side-effects when applied topically in that irritant-type contact dermatitis may develop *(*Rivers JK and McLean DI. An open study to assess the efficacy and safety of topical 3% diclofenac in a 2.5% hyaluronic acid gel for the treatment of actinic keratoses. Arch Dermatol 1997 October; 133(10):1239-42*).*

Thus, the findings of the present inventor are quite surprising because unlike other used NSAIDs, Oxaprozin is found very effective and safe in treating dermatological diseases. In considering the present invention and the treatment of inflamed tissue of the skin, Oxaprozin should no longer be regarded as a typical NSAID, because its principal pharmaco-dynamic properties relevant to inflamed skin do not include cyclooxygenase (Cox-1/Cox-2) inhibition. In fact, Oxaprozin is one of the NSAIDs with poorest Cox-2 inhibitory activity and selectivity for Cox-2 inhibition *(*Kawai S. Cyclooxygenase selectivity and the risk of gastrointestinal complications of various non-steroidal anti-inflammatory drugs. A clinical consideration. In Inflamma. Res. Supplement 2 (1998) S102-S106*).* Thus, the present inventor believes that Oxaprozin does not belong to the group of Cox-2 inhibitors.

The patent applications, US2005014729 and WO05009342 (both of Pharmacia Corporation) relate to methods for treating dermatological diseases, preferably acne, by administering a Cox-2 inhibitor. The technical teaching found herein concerns the treatment of skin diseases by administering a Cox-2 inhibitor. It is further taught that any compound having Cox-2 inhibition can be used and that such compounds preferably are selective Cox-2 inhibitors, but that NSAIDs can also be applied. An exhaustive list of NSAIDs is mentioned in US2005014729 and WO05009342 and includes Oxaprozin. However, the disclosures in US2005014729 and WO05009342 are unclear and ambiguous in nature and the applications fail to teach the specific utility of Oxaprozin for the treatment of skin diseases. Moreover, the invention as defined in US2005014729 and WO05009342 is very broadly defined and is more conceptual than substantial, in that all substances with Cox-2 inhibition are claimed to be effective in the treatment of all skin diseases despite the fact that some of them are already marketed for the treatment of a dermatological disease and others have failed to show effect in the treatment of dermatological diseases.

Moreover, the invention defined in US2005014729 and WO05009342 applications is so broadly defined and is more conceptual than substantial in that all substances with Cox-2 inhibition is claimed to be effective in the treatment of all skin diseases despite the fact that some of them are already marketed for the treatment of a dermatological disease and others have failed to show effect in the treatment of pruritus.

The following patent applications also relate to the treatment of dermatological diseases by administering various NSAIDs, but they all fail to directly and unambiguously disclose the use of Oxaprozin for the treatment of pruritus.

The patent application, JP7316075A2 (POLA CHEM IND INC) discloses dermatological formulations containing an antiphlogistic sedative drug and 10-20% of a water-retaining agent. The formulations are intended for the treatment of dry skin or for the treatment of dry skin associated with a skin disease, such as atopic dermatitis. As antiphlogistic sedative drugs are suggested NSAIDS in general including Oxaprozin with emphasis on parfenac (bufexamac), indomethacin, ibuprofen and tenoxicam; steroids, such as dexamethasone, clobetasone, prednisolone and hydrocortisone; and vitamin A derivatives, such as tocoretinate. Thus, according to the invention of JP7316075A2 a very large group of antiphlogistic sedative drugs are suggested despite the fact that their pharmacological properties are very different and unrelated in nature. The application fails to clearly direct the skilled person towards utilizing Oxaprozin for the treatment of the inflammation in a skin disease.

US2005232957A1 of Katz K (published 20-10-2005) relates to pharmaceutical compositions for the treatment of dry skin, such as in a subject suffering from atopic dermatitis and contact dermatitis. The compositions comprise specific (such as rofecoxib) or non-specific Cox inhibitors (an exhaustive list of Cox inhibitors including Oxaprozin is mentioned). The pharmacological principle of the invention is based on the expectation that Cox inhibitors enhances the concentration of sodium chloride in sweat and/or the water-binding capacity of keratins in the epidermis and thereby moistures the skin.

WO9735573A2 (THE BOOTS COMPANY PLC) relates to the treatment of pruritus by administering one or more NSAIDs selected from bendazac, benzydamine, diclofenac, fenbufen, indomethacin, ketoprofen, naproxen, piroxicam and sulindac.

WO9511017A1 (THE BOOTS COMPANY PLC) relates to the treatment of pruritus by administering ibuprofen or flurbiprofen.

WO02074290 (AGIS INDUSTRIES (1983) LTD) relates to a method of treating the skin disease, rosacea, by topical administration of a nonsteroidal anti-inflammatory drug (NSAID).

Thus, the present invention provides an effective and safe treatment of pruritus in general and pruritus associated with inflammatory or allergic dermatological diseases including treating the underlying disease causing the pruritus. This is quite surprising because many attempts have been made over time to apply NSAIDs in the treatment of dermatological diseases and pruritus, but the success has been limited so far because of too low effect and significant skin irritation.

### SUMMARY OF THE INVENTION

Surprisingly, the present inventor has found that Oxaprozin possess a strong therapeutic potential in the general management of pruritus as well as the treatment of some of the underlying diseases causing pruritus. Clinical data shown herein clearly demonstrates the significant immediate and complete alleviation of pruritus in patients suffering from contact dermatitis, atopic dermatitis, psoriasis and insect bite inflammation. Furthermore, erythema and scaling were also improved during the first 1-2 weeks of the treatment of contact dermatitis, atopic dermatitis and psoriasis indicating a therapeutic effect on the underlying disease causing the pruritus. Oxaprozin displayed a significantly strong inhibiting effect comparable to that of a strong steroid betamethasone-17-valerate at clinically relevant doses.

The present inventor puts forward the hypothesis that the convincing anti-pruritus effect observed with Oxaprozin is related to other pharmaco-dynamic effects than its inhibitory effect on prostaglandin synthesis.

The present inventor puts forward the hypothesis that the convincing effect observed with Oxaprozin is associated with the excellent pharmacological properties of Oxaprozin, namely the inhibition, in micromolar concentrations of one or more of the enzymes; Protein tyrosine kinases Syk, Protein tyrosine kinases ZAP-70 and PDE-IV enzyme. Such micromolar concentrations of Oxaprozin are expected to be present in skin cells following topical administration but pharmacologically active doses are also expected to be present following systemic administration.

Contrary to existing therapeutic agents used for treating inflammatory dermatological diseases, such as eczemas, the treatments according to the present invention have the advantage of not being likely to be associated with any serious side effects, as Oxaprozin has been shown to be safe and well tolerated by the organism in pharmacologically relevant doses. For example, Oxaprozin (in the form of its monoethanolamine salt) does not produce any cutaneous side-effects in the form of sensitization, phototoxic reaction, or acute dermal irritation.

Accordingly, in a first aspect the present invention provides the use of Oxaprozin or a closely related compound or a pharmaceutically acceptable salt thereof for the preparation of a medicament formulated for systemic administration or topical administration to skin for the treatment or prevention of pruritus in skin.

In various aspects of the invention pruritus is associated with:
■ a dermatological disease, such as an inflammatory or allergic dermatological disease
■ hypersensitivity reaction
■ type-IV allergy reaction in skin,
■ type-I allergy reaction in skin,
■ insect bite inflammation, bullous pemphigoid, cutaneous T-cell lymphoma, dermatitis herpetiformis, folliculitis, lichen planus, lichen simplex chronicus, pediculosis, prurigo nodularis, scabies, sunburn and urticaria,
■ asteatotic eczema, senile pruritus, stasis dermatitis, psoriasis, seborrheic dermatitis and seborrhea,
■ systemic medications,
■ exposure to water, or
■ systemic disease or organ failure

In still further aspects, the invention provides the use of Oxaprozin or a closely related compound or a pharmaceutically acceptable salt thereof for the preparation of a medicament formulated for systemic or topical administration to skin for the treatment or prevention of a dermatological disease where pruritus is a symptom of the skin.

In various aspects thereof, the treatment or prevention relates to the treatment or prevention of one or more of the symptoms selected from the group consisting of pruritus, erythema, oedema and scaling in a subject having a dermatological disease.

### DETAILED DESCRIPTION OF THE INVENTION

The invention relates to the treatment or prevention of pruritus in general or pruritus associated with a disease.

One aspect of the invention relates to to the use of Oxaprozin or a closely related compound or a pharmaceutically acceptable salt thereof for the preparation of a medicament formulated for systemic administration or for topical administration to skin for the treatment or prevention of pruritus in skin.

In the context of the present invention, the term "pruritus" is meant to be interchangeable with the term "itch" and defines a well known sensory state associated with the desire to scratch. The sensory state associated with pruritus is different from that of pain although pruritus and pain can be produced by a variety of chemical, mechanical, thermal or electrical stimuli. Itch and pain differ in that (1) itch, unlike pain, can only be evoked from the superficial layers of skin, mucosa, and conjunctiva, and (2) itch and pain usually do not occur simultaneously from the same skin region. For example, the application of histamine to skin produces itch but not pain. Furthermore, itch and pain are treated by different pharmacologically principles in that itch appears to be insensitive to opiate and non-steroidal anti-inflammatory drug (NSAID) treatment, both of which are effective in treating pain. Finally, itch occurs only in the skin; pain arises from deeper structures as well. Pruritus may be localised in various well defined areas of the skin, such as skin of the ankle, wrest, lips, hands, chest and the like, or it might be general pruritus not localised in a particular part of the skin.

It is anticipated that Oxaprozin or the closely related compound may be effective in the treatment of pruritus caused by a plethora of conditions, e.g. relevant to multiple types of pruritus as classified by Twycross et al. (Quart. J. Med. 2003; 96:7-26), namely *pruritoceptive* (cutaneous, e.g. scabies), *neuropathic* (due to lesions of afferent pathways of the nervous system, e.g. peripheral neuritis, brain tumours), *neurogenic* (due to centrally acting mediators which do not damage the central nervous system, e.g. opioid peptides of cholestasis) and *psychogenic.*

Particularly, it is anticipated that Oxaprozin or a closely related compound is effective in the treatment of pruritus associated with and/or caused by dermatological diseases; systemic disorders; organ failure; or use of various drugs.

In one particular aspect, pruritus is associated with an inflammatory dermatological disease or an allergic dermatological disease.

### The following definitions are used herein:

The phrase "closely related compound" is meant to define compounds resembling Oxaprozin in its molecular structure and which are further defined below.

In the context of the present invention, the term "treatment of pruritus associated with and/or caused by dermatological diseases" is meant to define the treatment of pruritus in a dermatological disease, where at least one of the enzymes selected from the group consisting of Protein tyrosine kinase Syk; Protein tyrosine kinase ZAP-70 and PDE-IV enzyme play a role in mediating the dermatological disease.

The phrase "where at least one of the enzymes selected from the group consisting of Protein tyrosine kinase Syk; Protein tyrosine kinase ZAP-70 and PDE-IV enzyme play a role in mediating the dermatological disease" is meant to define various dermatological diseases where over-expression or excessive amounts of these enzymes play a role. The following dermatological diseases are meant as non-limiting examples of such diseases:
acne vulgaris, adult eczema, alopecia, allergic contact dermatitis, allergic dermatitis, allergic contact eczema, asteatotic eczema, atopic eczema, hand eczema, atopic dermatitis, carcinomas, childhood eczema, chronic dermatitis of hands or feet, contact dermatitis, contact eczema, discoid eczema, insect bite inflammation, drug-induced skin reactions, dermatitis herpetiformis, discoid lupus erythematosus, eczema, epidermolysis bullosa, erythroderma, erythema nodosum, erythema multiforme, hand eczema, hand and
foot dermatitis, ichthyosis vulgaris, infantile eczema, keratoconus, keratosis pilaris lichen simplex chronicus, lichen planus, nummular dermatitis, melanomas, over-treatment dermatitis, pemphigus, pemphigoid, photodermatoses, pityriasis rosea, pyoderma gangrenosum, pompholyx, psoriasis, prurigo nodularis, rosacea, scabies, seborrheic dermatitis, seborrhea, scleroderma, Sjogren's Disease, stasis dermatitis, subacute cutaneous lupus erythematosus, sunburn, cutaneous manifestations of systemic lupus erythematosus, vitiligo, urticaria and xerotic eczema.

The term "skin cells" is meant to encompass cells present in stratum corneum, dermis and epidermis. When considering inflammatory dermatological diseases, such cells may include cells that constitute the inflammation in skin, such as T-cells, macrophages, mast cells, Langerhans cells and neutrophils.

The term "skin" is meant to include skin of the entire embody including the scalp, the forehead, the head, arms, legs, breast and so forth. The term "skin" is also meant to include various layers of the skin, such as stratum corneum, epidermis and dermis.

The term "subject" for purposes of treatment includes any subject, but is preferably a subject who is in need of the treatment of an inflammatory dermatological disease. For purposes of prevention, the subject is any subject, and preferably a subject that is at risk of, or is predisposed to, developing an inflammatory dermatological disease. The subject is typically an animal, and yet more typically is a mammal. "Mammal", as used herein, refers to any animal classified as a mammal, including humans, domestic and farm animals, zoo, sports, or pet animals, such as dogs, horses, cats, cattle, etc. Preferably, the mammal is a human. Typically, a subject is a human diagnosed or suffering from various forms of eczemas, such as contact dermatitis, atopic dermatitis and hand eczemas. In preferred embodiments the subject is a human, a dog, a cat or a horse.

The terms "treat", "treating" and "treatment" as used herein are meant to include alleviating or abrogating an inflammatory dermatological disease or its attendant symptoms and alleviating or eradicating the cause of the disease itself.

The terms "prevent", "preventing" and 'prevention", as used herein, refer to a method of delaying or precluding the onset of symptoms of an inflammatory dermatological disease, such as preventing the reoccurrence of inflammation or pruritus.

The term "therapeutically effective amount" refers to the amount of Oxaprozin or a related compound that will elicit the biological or medical response of a tissue, system, animal or human that is being sought by the researcher, veterinarian, medical doctor or other clinician. The term "therapeutically effective amount" includes that amount of a compound that, when administered, is sufficient to prevent development of, or alleviate to some extent, pruritus being treated. The therapeutically effective amount will vary depending on the compound, the disease and its severity and the age, weight, etc., of the mammal to be treated. With respect to topical administration of an effective amount of Oxaprozin or a closely related compound or a salt thereof to skin, it is considered to apply a dermatological formulation comprising between 0.1% to 10% by weight of Oxaprozin or a closely related compound or a salt thereof to the affected skin areas for 1 to 4 times daily. In methods or uses of the invention where Oxaprozin or a derivative thereof is administered systemically, a daily dose level of 1-200 mg/(kg body weight) is applied depending on the duration of the treatment, the condition to be treated, the formulation and the bioavailability. In a preferred embodiment of the invention the daily dose level is 5-100 mg/(kg body weight). In an even more preferred embodiment of the invention the daily dose level is 10-50 mg/(kg body weight).

The phrase "formulated for topical administration to skin" and topical administration is meant to define interchangeable terms that encompasses the formulation of the active ingredient of this invention (Oxaprozin or a closely related compound) into a dosage form that can be applied to skin of a subject and which result in the local presence of the active ingredient in the skin. The phrase "local presence of the active ingredient in skin" is meant to include topical administration of the active ingredient to skin with the presumption that systemic uptake of the active ingredient is limited or nil. Thus, it is intended that less than 25% by weight, such as less than 20% by weight, such as less than 15% by weight, such as less than 10%, 8%, 5% and 3% by weight, of the topically administered active ingredient enters the blood stream or is recovered in urine and faeces. Dosage forms for topical application to skin typically encompass emulsions (creams), ointments, gels, liniments, powders and solutions.

The phrase "formulated for systemic administration" is meant to define the formulation of the active ingredient of this invention (Oxaprozin or a closely related compound) into a dosage form, which when administered to a subject results in the systemic uptake of the active ingredient into the blood. The phrase "systemic uptake of the active ingredient" or "systemic administration" is interchangeable terms and is meant to include any form of administration of the active ingredient resulting in the entrance of the active ingredient into the blood stream. Therefore, the active ingredient may be administered by the per-oral, transdermal, transmucosal or the parenteral route, preferably by the oral route.

Generally spoken pruritus may be associated with a plethora of dermatological diseases, irrespective of their nature or to what extent inflammation or hypersensitivity reactions are part of the pathology. Non-limiting examples on such dermatological diseases are: acne vulgaris, alopecia, asteatotic eczema, melanomas, insect bite inflammation, drug-induced skin reactions, dermatitis herpetiformis, discoid lupus erythematosus, epidermolysis bullosa, erythroderma, erythema nodosum, erythema multiforme, lichen simplex chronicus, lichen planus, pemphigus, pemphigoid, photodermatoses, pityriasis rosea, pyoderma gangrenosum, pompholyx, psoriasis, prurigo nodularis, rosacea, scabies, seborrheic dermatitis, seborrhea, scleroderma, Sjogren's Disease, stasis dermatitis, subacute cutaneous lupus erythematosus, sunburn, cutaneous manifestations of systemic lupus erythematosus, vitiligo, and urticaria.

Further examples are those associated with atopic dermatitis including varies forms thereof and contact dermatitis including varies forms thereof.

Dermatological causes of pruritus often relates to hypersensitivity reactions in skin or allergic reactions, such a type-I or type-IV allergy reactions in skin. Thus, pruritus may be associated with atopic dermatitis and the various types thereof (atopic dermatitis, hand eczema, infantile eczema, childhood eczema, adult eczema, keratosis pilaris, ichthyosis vulgaris, hand and foot dermatitis, keratoconus, pompholyx, discoid eczema, nummular eczema) and allergic contact reactions, such as with contact dermatitis and the various types thereof (allergic contact dermatitis, irritant contact dermatitis and over-treatment dermatitis).

Typically, pruritus is an unpleasant symptom of insect bites and stings, bullous pemphigoid, cutaneous T-cell lymphoma, dermatitis herpetiformis, folliculitis, lichen planus, lichen simplex chronicus, pediculosis (lice infestation), prurigo nodularis, psoriasis, scabies, sunburn, urticaria and xerotic eczema.

While the treatment of pruritus in eczemas is one of the objects in another patent application of the same inventor, a preferred embodiment of this invention relates to the treatment of pruritus that is not directly associated with eczemas, but to the treatment of pruritus associated with other dermatological diseases, such as those mentioned below.

Therefore, in one preferred embodiment of the invention, the treatment of pruritus in skin is associated with or caused by insect bites (such as insect bite inflammation), insect stings, bullous pemphigoid, cutaneous T-cell lymphoma, dermatitis herpetiformis, folliculitis, lichen planus, lichen simplex chronicus, pediculosis (lice infestation), prurigo nodularis, scabies, sunburn, and urticaria.

Insect bite inflammation refers to the inflammation and/or allergic reaction caused by the insect bites of skin caused by for example mosquitoes, sand flies, fleas and the like.

Bullous pemphigoid is a dermatological disease presenting initially pruritic urticarial lesions. Tense blisters are produced after urticaria.

Cutaneous T-cell lymphoma (mycosis fungoides) is a dermatological disease presenting oval eczematous patch on skin with no sun exposure (e.g., buttocks). Possible presentation as new eczematous dermatitis in older adults. Possible presentation as erythroderma (exfoliative dermatitis).

Dermatitis herpetiformis is a dermatological disease affecting lumbosacral spine, elbows, or knees.

Folliculitis is a dermatological disease presenting pruritus out of proportion to appearance of dermatitis.

Lichen planus is a dermatological disease presenting lesions often located on the flexor wrists.

Lichen simplex chronicus refers to eczema that is a reaction to repeatedly scratching or rubbing of the skin in one location. A nervous scratching habit can lead to thickened, discoloured skin on the wrist, ankle, groin or back of the neck. Skin picking can lead to smaller bumps of the same type of rash called prurigo nodularis.

Pediculosis (lice infestation) is a dermatological disease caused by lice.

Prurigo nodularis refer to a skin condition in which hard crusty lumps form on the skin that itches intensely, some times constantly and mostly at night.

Scabies is a dermatological disease presenting burrows in hand web spaces, axillae, and genitalia, hyperkeratotic plaques, pruritic papules, or scales.

Sunburn is a dermatological disease possible caused by photosensitizing (e.g., to nonsteroidal anti-inflammatory drugs and cosmetics)

Still other embodiments refer to the treatment or prevention of eczemas and dermatitis which do not typically involve a hypersensitivity reaction, such as asteatotic eczema including senile pruritus, stasis dermatitis, psoriasis, seborrheic dermatitis and seborrhea.

Asteatotic eczema (xerotic eczema) refers to eczema that dries the skin, causing fine cracks in the skin, usually first involving the lower legs, where there are fewer oil glands. It commonly occurs in the elderly, especially during winter months spent indoors in low humidity environments. Elderly may develop a condition termed senile pruritus.

Stasis dermatitis refers to eczema occurring on the calves, ankles and feet of people who have varicose veins or other conditions that lead to poor blood circulation in the lower legs, this type of dermatitis has leg swelling leads to itching, fine red bumps, skin darkening and, sometimes, ankle sores.

Psoriasis refers to a chronic inflammatory skin disease characterised by hyperproliferation of the epidermis. This disease is manifested by red plaques (erythema) covered with whitish flakes (scaling) which detach from the skin and pruritus is a serious unpleasant predominant symptom.

Seborrheic dermatitis may be considered as a type of eczema, although it creates a greasier rash than usual for eczema. This scaly dermatitis commonly appears on the scalp of infants (as cradle cap) or as dandruff in adults. Probably triggered by the skin fungus Pityrosporum ovale, it commonly affects the face or neck around the nose and at the scalp line.

Seborrhea is a condition characterized by excessive oiliness of the skin, especially of the scalp and face, but without the characteristic redness or scaling of Seborrheic dermatitis.

In still further embodiments of the invention pruritus is a symptom of:
1) heat exposure, such as resulting in cholinergic urticaria (response to hot bath, fever, exercise) and miliaria rubra (prickly heat);
2) occupational exposure, such as caused by fibreglass, glyceryl monothioglycolate, methyl methacrylate (e.g., plexiglas), potassium dichromate in cements and dyes, rosins or epoxy resins in adhesives and rubber;
3) systemic medications such as with antifungal agents like fluconazole (Diflucan), itraconazole (Sporanox), ketoconazole (Nizoral), Aspirin, B vitamins, including niacinamide, drug hypersensitivity to rifampin (Rifadin), vancomycin (Vancocin), nitrates (food preservatives), quinidine and spinal narcotics (pruritus affecting face, neck, and upper chest);
4) water exposure, such as resulting in aquagenic pruritus (associated with polycythemia vera, itching within 15 minutes of any water contact), cholinergic urticaria (response to warm water), polycythemia vera, swimmer's itch (seven-day eruption after freshwater swimming).

Thus, in other relevant embodiments of the invention pruritus is associated with systemic medications and water exposure.

Furthermore, pruritus can be caused by an underlying systemic disease. A wide variety of systemic diseases can cause generalized pruritus without diagnostic skin lesions. Typical examples of systemic diseases causing pruritus are:
- Infections with bacteria, virus, funghi and parasites such as tropical and intestinal parasites (Rubella, Varicella, Trichinosis, Onchocerciasis, Schistosomiasis)
- Endocrine diseases such as diabetes, hyperthyroidism, hypothyroidism, disorders of the parathyroid gland, carcinoid syndrome, hepatic disease, pregnancy, intrahepatic cholestasis, obstructive jaundice (in biliary tract or extrahepatic), primary biliary cirrhosis, drug induced cholestasis,
- Renal diseases such as chronic renal failure and uraemia.
- Haematological diseases such as polycythaemia vera, iron deficiency, Hodgkin's Disease, Mycosis fungoides, Lymphosarcoma, Chronic leukaemia, Myleomatosis, Paraproteinaemia, Mast cell disease, HIV, Sezary's syndrome (T-cell lymphoma), leukaemia, multiple myeloma, Waldenström's macroglobinaemia, mycosis fungoides, benign gammopathy, systemic mastocytosis;
- Occult malignancy, such as haematological and lymphoproliferative disorders, carcinomatosis, adenocarcinoma and squamous cell carcinoma of various organ, tumor of brain;
- Neurological diseases such as multiple sclerosis, brain tumor;
- Psychiatric/Psychogenic causes such as emotional stress and psychological trauma;
- Drugs such as opium alkaloid, CNS stimulant/depressant, niacinamide, cimetidine, aspirin, quinidine, chloroquine.

Typical examples on systemic diseases causing pruritus and which are embodiments of the present invention are: diabetes, hyperthyroidism, hypothyroidism, disorders of the parathyroid gland, carcinoid syndrome, hepatic disease, pregnancy, intrahepatic cholestasis, obstructive jaundice (in biliary tract or extrahepatic), primary biliary cirrhosis, drug induced cholestasis, chronic renal failure, uraemia, polycythaemia vera, iron deficiency, Hodgkin's Disease, Mycosis fungoides, Lymphosarcoma, Chronic leukaemia, Myleomatosis, Paraproteinaemia, Mast cell disease, HIV, Sezary's syndrome (T-cell lymphoma), leukaemia, multiple myeloma, Waldenström's macroglobinaemia, mycosis fungoides, benign gammopathy, systemic mastocytosis, haematological and lymphoproliferative disorders,carcinomatosis, adenocarcinoma and squamous cell carcinoma of various organ, tumour of brain, multiple sclerosis and brain tumours.

It should be understood that in some embodiments of the invention, the Oxaprozin or a closely related compound or a pharmaceutically acceptable salt thereof effectively treats more than one symptom of a dermatological disease, such as treating or preventing one or more of the symptoms selected from the group consisting of pruritus, erythema, oedema and scaling in a subject. That is to say that the underlying disease is also treated.

Therefore, another aspect of the invention relates to the use of Oxaprozin or a closely related compound or a pharmaceutically acceptable salt thereof for the preparation of a medicament formulated for systemic or topical administration to skin for the treatment of a dermatological disease where pruritus is a symptom.

Thus, the invention also relates to a method for the treatment or prevention of a dermatological disease where pruritus is a symptom comprising topical administering or systemic to skin an effective amount of Oxaprozin or a closely related compound or a pharmaceutically acceptable salt thereof to a subject in need thereof.

Non-limiting examples are acne vulgaris, adult eczema, alopecia, allergic contact dermatitis, allergic dermatitis, allergic contact eczema, asteatotic eczema, atopic eczema, hand eczema, atopic dermatitis, carcinomas, childhood eczema, chronic dermatitis of hands or feet, contact dermatitis, contact eczema, discoid eczema, insect bite inflammation, drug-induced skin reactions, dermatitis herpetiformis, discoid lupus erythematosus, eczema, epidermolysis bullosa, erythroderma, erythema nodosum, erythema multiforme, hand eczema, hand and foot dermatitis, ichthyosis vulgaris, infantile eczema, keratoconus, keratosis pilaris lichen simplex chronicus, lichen planus, nummular dermatitis, melanomas, over-treatment dermatitis, pemphigus, pemphigoid, photodermatoses, pityriasis rosea, pyoderma gangrenosum, pompholyx, psoriasis, prurigo nodularis, rosacea, scabies, seborrheic dermatitis, seborrhea, scleroderma, Sjogren's Disease, stasis dermatitis, subacute cutaneous lupus erythematosus, sunburn, cutaneous manifestations of systemic lupus erythematosus, vitiligo and urticaria.

In one further aspect, the invention relates to the use of use of Oxaprozin or a closely related compound or a pharmaceutically acceptable salt thereof for the preparation of a medicament formulated for systemic or topical administration to skin for the treatment or prevention of one or more of the symptoms selected from the group consisting of pruritus, erythema, oedema and scaling in a subject having a dermatological disease, such as psoriasis or seborrheic dermatitis.

### Oxaprozin and closely related compounds

As mentioned, in currently interesting embodiments of the invention, the inhibitor of the Protein Kinase SYK and the Protein Kinase ZAP-70 and/or PDE-IV enzyme is Oxaprozin or a salt thereof.

Oxaprozin is chemically designated 4,5-diphenyl-2-oxazole-propionic acid, and has the following chemical structure:

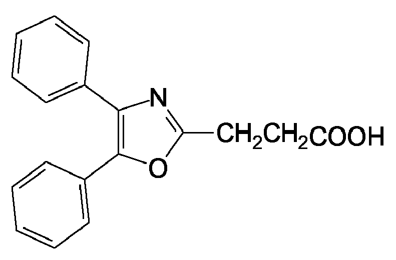

It should be understood that in a preferred embodiment of the invention, Oxaprozin is applied in un-derivatized form or as a pharmaceutically acceptable salt thereof or as a hydrolysable ester or amide.

It is anticipated that the novel pharmaco-dynamic effect of Oxaprozin is also exhibited by structurally closely related compounds and bioisosters of Oxaprozin. Several of such compounds with anti-inflammatory effect have been described in the patent literature. General derivatives as well as the manufacturing thereof are described in US 3,578,671. Specific derivatives and the manufacturing thereof are described in US 5,380,738 (4-fluorophenyl and 4-methylsulfonylphenyl), US 4,659,728 (hydroxy substituted derivatives), US 6,090,834 (sulfonyl derivatives), US 3,506679 (4,5-diarylthiazol derivatives).

Therefore as used herein, the term "closely related compound" includes compounds with the general formula I: 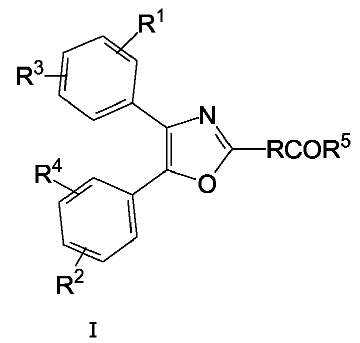 and bioisosters thereof.

Derivatives of Oxaprozin include various length of the R carbon chain implying that the propionic acid of the Oxaprozin molecule being replaced with acetic acid or butyric acid. The acid group may be derivatized into amides and esters, preferably into hydrolysable amides and esters that upon administration to a human or animal, are capable of providing (directly or indirectly) Oxaprozin or an active metabolite or residue thereof or a derivative of Oxaprozin, as defined herein. Furthermore, the R chain and the two phenyl rings may be subject to substitution by replacing one or more hydrogen(s) with substituents defined herein. Finally, the term "derivatives thereof" include bioisosters where the oxygen of the oxazole ring is replaced with sulfur (S) to provide a thiazole ring. A bioisoster may also be provided by replacing the carboxylic acid group by a thioacid, optionally in the form of a thioester by the proper selection of R⁵.

As used herein, the group R primarily defines straight chained or branched, saturated or unsaturated aliphatic carbon chain containing 2 carbon atoms connected in one end to the 2-position of the oxazole ring and in the other end to COR⁵. Thus, the R chain in combination with the COR⁵ -group forms n-propionic, iso-propionic and propionenic acids, esters and amides thereof. In closely related embodiments, the group R defines straight chained or branched, saturated or unsaturated aliphatic radical containing 1 or 3 carbon atoms. Thus, acetic, acrylic and butyric acids, esters and amides thereof are also anticipated.

Thus, R may be selected from C₁₋₃-alkyl, C₂₋₃-alkenyl, and C₂₋₃-alkynyl. The groups C₁₋₃-alkyl, C₂₋₃-alkenyl, and C₂₋₃-alkynyl may optionally be derivatized by substitution of one hydrogen atom with cyano (CN), halogen (Br, Cl. F, I), hydroxy (OH), amino (NH₂). or nitro (NO₂).

In preferred embodiments, the R group constitutes together with the COR⁵ a free acid side chain (where R⁵ is hydroxy (OH) or sulfhydryl (SH) such as in the form of acetic acid, acrylic acid, propionic acid, butyric acid including unsaturated, geometric and stereo isomers thereof. However, in other embodiments, the free acid (R⁵ is OH or SH) is converted into suitable esters (R⁵ is OR⁶ or SR⁶) or amides (R⁵ is amino (NH₂). primary amino (NHR') or secondary amino (NR'R")).

Thus, R⁵ may be selected from OH, OR⁶, NH₂, NHR', NR'R", SH or SR⁶.

R⁶ designates a radical selected from C₁₋₆-alkyl, C₂₋₆-alkenyl and aryl.

R' and R" define the same or different group selected from C₁₋₆-alkyl and C₂₋₆-alkenyl.

The term "aryl" means phenyl, mono- or di-substituted phenyl wherein one or two hydrogens have been replaced by substituents selected from C₁₋₆-alkyl, C₂₋₆-alkenyl, C₂₋₆-alkynyl, C₁₋₆-alkoxyl, carboxy (CO), carboxyderivative (COR'), carboxyaldehyde (CHO), carboxylic acid (COOH), carboxylderivative (COOR') cyano (CN), halogen (Br, Cl, F, I), hydroxy (OH), amino (NH₂), primary amino (NHR'), secondary amino (NR'R") and nitro (NO₂). Preferably, the term "aryl" means phenyl or mono-substituted phenyl wherein one hydrogen have been replaced by substituents selected from C₁₋₆-alkyl, C₂₋₆₋alkenyl, C₁₋₆-alkoxyl, CO, CHO, CN, halogen, OH, NH₂ and NO₂.

The terms "R¹, R², R³ and R⁴" are meant to define substituents of the phenyl rings of formula I so as to define un-substituted, mono-substituted or di-substituted phenyl rings, wherein R¹, R², R³ and R⁴ may be the same or different. The phrase "mono and di-substituted phenyl radicals" designates substitution of one or two hydrogen(s) in each phenyl ring with R¹ and/or R³ in one ring and independently thereof with R² and/or R⁴ in the second phenyl ring.

R¹ and R² independently designates radicals selected from hydrido, C₁₋₆-alkyl, C₂₋₆₋alkenyl, C₂₋₆-alkynyl, C₁₋₆-alkoxyl, carboxy (CO), carboxyaldehyde (CHO), carboxylic acid (COOH) and derivative thereof (CO-Me, CO-Et), cyano (CN), halogen (Br, Cl, F, I), hydroxy (OH), hydroxy derivative (OR'), amino (NH₂), primary amino (NHR'), secondary amino (NR'R"), nitro (NO₂), sulfonyl (HSO₂), sulfonyl derivative (R⁷-SO₂,), wherein R⁵, R⁶, R' and R" are as defined above. R⁷ designates a substituent selected from C₁₋₆-alkyl, aryl, NH₂, NHR', NR'R", wherein aryl and R' and R" are as defined above.

R³ and R⁴ independently designates radicals selected from hydrido, C₁₋₆-alkyl, C₂₋₆₋alkenyl, C₂₋₆-alkynyl, C₁₋₆-alkoxyl, CO, CHO, CO-Me, CO-Et, CN, COR⁵, halogen, OH, OR', NH₂, NHR', NR'R" and NO₂, wherein R⁵, R⁶, R' and R" are as defined above. In a preferred embodiment, R³ and R⁴ independently designate radicals selected from hydrido, C₁₋₆-alkyl and C₂₋₆-alkenyl. In a still more preferred embodiment, R³ and R⁴ each designate hydrido.

In the present context C₁₋₃-alkyl is designated to define straight chained or branched carbon chain having from 1 to 3 carbon atoms and wherein the carbon chain is situated between the oxazole ring and the COOR⁵ group, such as -CH₂-, -CH₂CH₂- and -CH₂CH₂CH₂-, including isomers thereof. Likewise, C₂₋₃-alkenyl and C₂₋₃-alkynyl means unsaturated aliphatic carbon chain containing 2 or 3 carbon atoms, such as -CHCH-, - CC-, -CHCHCH₂-, -CCCH₂-, including isomers thereof.

C₁₋₆-alkyl is meant to define saturated, straight chained or branched alkyl radical containing the number of carbon atoms indicated, e.g. "1-6" means all alkyl radicals from methyl up to hexyl including all isomers thereof, e.g. iso-butenyl. Where applicable, the alkyl may be on cyclical form, such as cyclohexane.

C₂₋₆-alkenyl defines unsaturated straight chained or branched alkylene radicals containing the number of carbon atoms indicated e.g. 1- or 2-propenyl, 1-, 2- or 3-butenyl and the like and isomers thereof.

C₂₋₃-alkynyl defines unsaturated chained or branched alkynyl radicals containing the number of carbon atoms indicated, e.g. ethynyl, 1- or 2-propynyl and isomers thereof.

C₁₋₆-alkoxyl means alkoxy radicals containing up to 6 and preferably up to 4 carbon atoms, e.g. methoxy, ethoxy, propoxy etc.

The groups, C₁₋₆-alkyl, C₂₋₆-alkenyl, may optionally be mono-substituted with CN, CO, CHO, COR⁵, halogen, OH, OR' NH₂, NHR', NR'R" and nitro, wherein R⁵, R⁶, R' and R" are as defined above.

The term halogen defines bromine, chlorine, fluorine and iodine. The term "hydrido" designates a single hydrogen atom (H).

The Oxaprozin derivatives of the present invention may contain asymmetric carbon atoms, and, therefore, the instant invention may also include the individual diastereomers and enantiomers, which may be prepared or isolated by methods known to those skilled in the art.

As mentioned, in current interesting embodiments of the invention Oxaprozin or a pharmaceutically acceptable salt is the therapeutically active ingredient.

In other interesting embodiments, the therapeutically active ingredient is a closely related compound according to formula I. In one group of embodiments designated A, R is -CH₂-. In another group of embodiments designated B, R is selected from C₂₋alkyl, C₂-alkenyl, and C₂-alkynyl, such as -CH₂CH₂-, -CHCH-, -CC-. In still another group of embodiments (designated C), R is selected from C₃-alkyl, C₃-alkenyl, and C₃₋alkynyl, such as -CH₂CH₂CH₂-, -CHCHCH₂-, -CCCH₂- and geometric and stereo isomers thereof. In all such embodiments (A, B and C), R may be substituted in that one hydrogen atom is replaced with CN, halogen, OH, NH₂, NO₂, preferably with OH. Furthermore, in such embodiments, R¹, R², R³, R⁴, R⁵, R⁶, R⁷, R' and R" are as defined above.

In further interesting embodiments of A, B and C (designated AA, BA, CA), R² and R⁴ independently designates radicals selected from hydrido, C₁₋₆-alkyl, halogen, OH and OR' and R¹ R³, R⁵, R⁶, R⁷, R' and R" are as defined above.

In other further interesting embodiments of A, B and C (designated AB, BB and CB), R² and R⁴ is hydrido and R¹, R³, R⁵, R⁶, R⁷, R' and R" are as defined above.

In further interesting embodiments of A, AA, AB, B, BA, BB, C, CA and CB, the groups R¹, R², R³, R⁴, R⁵, R⁶, R⁷, R' and R" are as defined under the respective groups of embodiments, but the term "aryl" is meant to designate phenyl or mono substituted phenyl, wherein one hydrogen has been replaced by substituents selected from C₁₋₆₋alkyl, C₂₋₆-alkenyl, C₂₋₆-alkynyl, C₁₋₆-alkoxyl, CN, CO, CHO, COOH, halogen, OH, NH₂, NHR', NR'R" and NO₂.

In still further interesting embodiments of A, AA, AB, B, BA, BB, C, CA and CB, the groups R¹, R², R³, R⁴, R⁵, R⁶, R⁷, R' and R" are as defined under the respective groups of embodiments, but the term "aryl" is meant to designate phenyl or mono substituted phenyl, wherein one hydrogen has been replaced by substituents selected from C₁₋₆₋alkyl, C₁₋₆-alkoxyl, CN, CHO, COOH, halogen, OH, NH₂, and NO₂.

In still further interesting embodiments of A, AA, AB, B, BA, BB, C, CA and CB, the groups R² and R⁴ independently designates radicals selected from hydrido, C₁₋₆-alkyl, C₁₋₆-alkoxyl, CN, COOH, halogen, OH, NH₂, NHR, NR'R", NO₂, HSO₂, R⁷-SO₂ and R¹, R³ , R⁵, R⁶, R⁷ R' and R" are as defined under the respective embodiments. In such embodiments, the term "aryl" is meant to designate phenyl or mono substituted phenyl, wherein one hydrogen has been replaced by substituents selected from C₁₋₆₋alkyl, C₁₋₆-alkoxyl, CN, CHO, COOH, halogen, OH, NH₂, and NO₂.

In still further interesting embodiments of A, AA, AB, B, BA, BB, C, CA and CB, the groups, C₁₋₆-alkyl, C₁₋₉-alkyl, C₂₋₆-alkenyl, C₂₋₉-alkenyl, C₂₋₆-alkynyl and C₂₋₉-alkynyl may optionally be mono-substituted with CN, halogen, OH, OR' NH₂, NHR', NR'R" and nitro and R¹, R², R³ , R⁴, R⁵, R⁶, R⁷ R' and R" are as defined under the respective embodiments. In such embodiments, the term "aryl" is meant to designate phenyl or mono substituted phenyl, wherein one hydrogen atom has been replaced by substituents selected from C₁₋₆-alkyl, C₁₋₆-alkoxyl, CN, CHO, COOH, halogen, OH, NH₂, and NO₂.

It should be understood that in still further interesting embodiments of the above all mentioned, R is -CH₂- or C₂-alkyl or C₂-alkenyl.

Typical examples on closely related compounds are:
4,5-diphenylthiazol-2-yl- propionic acid, optionally in the form of its ethyl or methyl ester;
4,5-diphenyloxazol-2-yl-acrylic acid;
4,5-diphenyloxazol-2-yl-acetic acid;
4,5-di-(4'-chlorophenyl)-oxazol-2-yl-propionic acid;
4,5-diphenyloxazol-2-yl)-propionamide;
4,5-diphenyloxazol-2-yl)-acrylic acid ethyl ester;
4-(4'-bromophenyl)-5-phenyloxazole-2-yl-propionic acid, optionally in the form of its methyl ester;
4-(4-hydroxyphenyl-5-phenyl-2-oxazole propanoic acid, optionally in the form of its ethyl or methyl ester;
4-(4-fluorophenyl)-5-[4-(methylsulfonyl)phenyl]-2-oxazolepropionic acid, optionally in the form of its methyl ester;
4-(4-fluorophenyl)-5-[4-(methylsulfonyl)-phenyl]-2-oxazoleacetic acid, optionally in the form of its ethyl ester;
4-(4-fluorophenyl)-5-[4-(methylsulfonyl)phenyl]-2-oxazolebutanoic acid, optionally in the form of its methyl ester;
4-(4-fluorophenyl)-5-(4-(methylsulfonyl)phenyl)-2-oxazolepropionic amide;
[4-(4-aminosulfonylphenyl)-5-(3,4-dichlorophenyl)]-2-oxazoleacetic acid, optionally in the form of its ethyl or methyl ester;
[4-(4-aminosulfonylphenyl)-5-(3-chloro-4-fluorophenyl)]-2-oxazoleacetic acid, optionally in the form of its ethyl or methyl ester;
[4-(4-aminosulfonylphenyl)-5-(3-fluoro-4-methoxyphenyl)]-2-oxazoleacetic acid, optionally in the form of its ethyl or methyl ester;
[4-(4-aminosulfonylphenyl)-5-(4-chlorophenyl)]-2-oxazoleacetic acid, optionally in the form of its ethyl or methyl ester;
[4-(4-aminosulfonylphenyl)-5-phenyl]-2-oxazolebutanoic acid, optionally in the form of its ethyl or methyl ester;
[4-(4-aminosulfonylphenyl)-5-phenyl]-2-oxazolepropanoic acid, optionally in the form of its ethyl or methyl ester;
[4-(4-aminosulfonylphenyl-5-(3,4-difluorophenyl)]-2-oxazoleacetic acid, optionally in the form of its ethyl or methyl ester;
[4-(4-methylsulfonylphenyl)-5-phenyl]-2-oxazoleacetic acid, optionally in the form of its ethyl or methyl ester;
[4-(4-methylsulfonylphenyl)-5-phenyl]-2-oxazolebutanoic acid, optionally in the form of its ethyl or methyl ester;
[4-(4-methylsulfonylphenyl)-5-phenyl]-2-oxazolepropanoic acid, optionally in the form of its ethyl or methyl ester;
4-[4-aminosulfonylphenyl)-5-(3-fluoro-4-methoxyphenyl)-2-oxazolyl].alpha.-bromoacetic acid, optionally in the form of its ethyl or methyl ester;
5-(4-nitrophenyl-4-phenyl-2-oxazole-2-yl propionic acid, optionally in the form of its ethyl or methyl ester;
5-(4'-fluorophenyl)-4-phenyloxazole-2-yl-propionic acid, optionally in the form of its methyl ester;
5-(4-hydroxyphenyl-4-phenyl-2-oxazole propanoic acid, optionally in the form of its ethyl or methyl ester;
5-(4-fluorophenyl)-4-[4-(methylsulfonyl) phenyl]-2-oxazolepropionic acid, optionally in the form of its ethyl or methyl ester;
[5-(4-aminosulfonylphenyl)-4-(4-chlorophenyl)]-2-oxazoleacetic acid, optionally in the form of its ethyl or methyl ester;
[5-(4-aminosulfonylphenyl)-4-phenyl]-2-oxazoleacetic acid, optionally in the form of its ethyl or methyl ester;
[5-(4-aminosulfonylphenyl)-4-phenyl]-2-oxazolebutanoic acid, optionally in the form of its ethyl or methyl ester;
[5-(4-aminosulfonylphenyl)-4-phenyl]-2-oxazolepropanoic acid, optionally in the form of its ethyl or methyl ester;
[5-(4-aminosulfonylphenyl)-4-phenyl]-2-oxazolepropionic acid, optionally in the form of its ethyl or methyl ester;
ethyl [4-(4-aminosulfonylphenyl)-5-(3-fluoro-4-methoxyphenyl)]-2-oxazoleacetate;
ethyl [4-(4-aminosulfonylphenyl)-5-phenyl]-2-oxazoleacetate;
ethyl [4-(4-aminosulfonylphenyl)-5-phenyl]-2-oxazolebutanoate;
ethyl [4-(4-aminosulfonylphenyl)-5-phenyl]-2-oxazolepropanoate;
ethyl [4-(4-methylsulfonylphenyl)-5-phenyl]-2-oxazoleacetate;
ethyl [4-(4-methylsulfonylphenyl)-5-phenyl]-2-oxazolebutanoate;
ethyl [4-(4-methylsulfonylphenyl)-5-phenyl]-2-oxazolepropanoate;
ethyl [5-(4-chlorophenyl) - 4-phenylthiazol] 2-yl propionic acid;
ethyl [5-(4-aminosulfonylphenyl)-4-phenyl]-2-oxazoleacetate;
ethyl [5-(4-aminosulfonylphenyl)-4-phenyl]-2-oxazolebutanoate;
ethyl [5-(4-aminosulfonylphenyl)-4-phenyl]-2-oxazolepropanoate;
methyl [4-(4-aminosulfonylphenyl)-5-phenyl]-2-oxazoleacetate;
methyl [4-(4-aminosulfonylphenyl)-5-phenyl]-2-oxazolebutanoate;
methyl [4-(4-aminosulfonylphenyl)-5-phenyl]-2-oxazolepropanoate;
methyl [4-(4-methylsulfonylphenyl)-5-phenyl]-2-oxazoleacetate;
methyl [4-(4-methylsulfonylphenyl)-5-phenyl]-2-oxazolebutanoate;
methyl [4-(4-methylsulfonylphenyl)-5-phenyl]-2-oxazolepropanoate;
methyl [5-(4-aminosulfonylphenyl)-4-phenyl]-2-oxazoleacetate;
methyl [5-(4-aminosulfonylphenyl)-4-phenyl]-2-oxazolebutanoate;
methyl [5-(4-aminosulfonylphenyl)-4-phenyl]-2-oxazolepropanoate.

In summary, Oxaprozin or a closely related compound is meant to include derivatives according to formula I, which include metabolites of Oxaprozin and suitable prodrugs thereof, bioisosters thereof and pharmaceutically acceptable salts thereof including a solvate of the salt. Typical examples on suitable esters are formiate, acetate, propionate, ascorbyl and benzoylate. Examples on metabolites of Oxaprozin are hydroxy substituted Oxaprozin, namely 5-(4-hydroxyphenyl)-4-phenyl-2-oxazolepropanoic acid, 4-(4-hydroxyphenyl)-5-phenyl-2-oxazolepropanoic acid and 4-(4-hydroxyphenyl)-5-(4-hydroxyphenyl)-2-oxazolepropanoic acid as described in US 4,659,728.

In considering providing dermatological formulations comprising high amounts of completely dissolved Oxaprozin or a closely related compound, the solubility of the actives needs to be improved. One means to that effect is to improve the solubility of Oxaprozin by forming a water-soluble salt of Oxaprozin or a closely related compound defined herein. Therefore, in some embodiments of the invention, optionally where R⁵ is hydroxy (OH), the Oxaprozin itself or a closely related compound may be provided as a pharmaceutically acceptable water-soluble salt.

The term "water-soluble" is meant to define Oxaprozin or a derivative thereof modified in a manner resulting in much higher water-solubility than Oxaprozin itself, such as 10, 20, 25, 40, 50, 75, 100, 200, 250, 400, 500, 750 and 1000 times higher. The solubility of Oxaprozin in water at 25°C is about 1.7 mg/ml. Therefore, a water-soluble modification of Oxaprozin or a derivative thereof is meant to denote a modification resulting in a solubility of the modified Oxaprozin or related compound in water at 25°C of at least 50 mg/ml, such as of at least 75, 100, 150, 200, 250 or even at least 300 mg/ml.

The term "closely related compound" is also meant to define a pharmaceutically acceptable salt of Oxaprozin or of the related compound. Thus, Oxaprozin and the closely related compound where R⁵ is hydroxy (OH) may be provided in the form of a single salt, either as a base addition salt or as an acid addition salt, or in the form of a double salt in the event where both the free carboxylic acid and the nitrogen of the oxazole ring form a salt.

In the present context, the phrase "a pharmaceutically acceptable salt" encompasses a base addition salt derived from the reaction of the free propionic acid entity with inorganic bases (hydroxides) or organic bases or/and an acid addition salt derived from the reaction of the basic oxazole ring nitrogen with pharmaceutically acceptable acids. Thus, it might be understood that Oxaprozin may be provided in the form of an acid addition salt or a base addition salt or in the form of a double salt of mixed acid addition and base addition salt.

Examples of base addition salts encompass Na, K, Ca, Mg, Cu, Zn and Mn salts. Typically organic bases for use in the preparation of a base addition salt are primary, secondary or tertiary amines including alkylphenylamine, ammonia, 2-aminoethanol, aminopyrimidine, aminopyridine, arginine, benethamine, benzathine, betaine, caffeine, choline, deanol, diethanolamine, diethylamine, 2-diethylaminoethanol, 2-dimethylaminoethanol, ethylenediamine, N- ethylmorpholine, N-ethylpiperidine, glucamine, glucosamine, glycinol, hydrabamine, imidazol, isopropylamine, meglumine, methylglucamine, morpholine, piperazine, piperidine, procaine, purine, pyrrolidine, theobromine, thiamine, triethanolamine, triethylamine, trimethylamine, tripropylamine, tromethamine, spermidine, and the like). Furthermore, base addition salts may be derived from the reaction with natural amino acids such as with glycine, alanine, valine, leucine, isoleucine, norleucine, tyrosine, cystine, cysteine, methionine, proline, hydroxy proline, histidine, omithine, lysine, arginine, serine, threonine, and phenylalanine and with unnatural amino acids such as D-isomers or substituted amino acids; guanidine, substituted guanidine wherein the substituents are selected from nitro, amino, alkyl, alkenyl, alkynyl, ammonium or substituted ammonium salts and aluminum salts.

Examples on acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic or phosphorous acids and the like, as well as the salts derived from relatively non-toxic organic acids like acetic, propionic, isobutyric, maleic, malonic, benzoic, succinic, suberic, fumaric, mandelic, phthalic, benzenesulfonic, p-tolylsulfonic, citric, tartaric, methanesulfonic, and the like. Also included are salts of amino acids such as arginate and the like, and salts of organic acids like glucuronic or galacturonic acids and the like.

Where Oxaprozin or a closely related compound is provided in the form of a salt it is meant to include a pharmaceutically acceptable solvate, which may be a hydrate (comprising from half a mole of H₂O up to about 10 moles of H₂O per mole of salt) or comprise other solvents of crystallization such as alcohols.

As mentioned, the active ingredient of the invention may be administered to a subject through any route of administration resulting in either local presence of the agonist in skin or systemic uptake.

In currently interesting embodiments of the invention, the Oxaprozin or a closely related compound is administered topically to the skin of a subject. That is to say that Oxaprozin or a medicament thereof is preferably formulated for topical application to the skin, such as formulated in liquid or semi- solid form (including, for example, ointment, emulsion including microemulsions and liposomes, gel, liniment, powder or spray) or it may be provided in combination with a "finite" carrier, for example, a non-spreading material that retains its form, including, for example, a patch, bioadhesive, dressing or bandage. The Oxaprozin or a closely related compound may be formulated in aqueous or non-aqueous form, such as a solution, emulsion, dispersion, suspension or ointment.

Topical administration refers to the application of a dermatological composition comprising Oxaprozin or a closely related compound in a concentration of 0.01 - 50.0 % (w/w). The amount of dermatological composition applied depends on the duration of the treatment, the condition to be treated and the formulation. In a preferred embodiment of the invention the concentration of Oxaprozin or a closely related compound in the dermatological composition is 0.1 - 20.0 % (w/w). In an even more preferred embodiment of the invention the concentration in the formulation is 0.5 - 10.0 % (w/w).

In considering applying more effective amounts of the active compounds of the invention, a medicament, such as a dermatological formulation, and methods of topical administration of a therapeutically effective amount should comprise Oxaprozin or a closely related compound in an amount of at least 0.5% by weight, more preferably of at least 1% by weight, even more preferably of at least 1.5% by weight, still more preferably of at least 2 % by weight, such as about 2.5% by weight, about 3% by weight, about 3.5% by weight, about 4% by weight, about 4.5% by weight, about 5% by weight, about 5.5% by weight, about 6% by weight or about 7% by weight.

Although Oxaprozin or a closely related compound is well tolerated in skin, it may be considered to apply amounts of the actives that secure safe treatment. Therefore, a medicament, such as a dermatological formulation, and methods of topical administration of a therapeutically effective amount should comprise Oxaprozin or a closely related compound in an amount less than 7% by weight, more preferably less than 6.5 % by weight, even more preferably less than 6% by weight, still more preferably less than 5.5 % by weight, even still more preferably less than 5% by weight, such as less than 4.5% by weight, such as less than 4% by weight, or such as less than 3.5% by weight.

Accordingly, in preferred embodiments of the invention, a medicament, such as a dermatological formulation, and methods of topical administration of a therapeutically effective amount should comprise Oxaprozin or a closely related compound or a salt thereof in an amount ranging between 0.5 and 10% by weight, such as between 0.5 and 8% by weight, preferably between 0.5 and 7% by weight, such as between 0.5 and 6% by weight, between 0.5 and 5.5 % by weight, between 0.5 and 5% by weight, between 0.5 and 4.5% by weight, between 0.5 and 4% by weight, between 0.5 and 3.5% by weight, such as between 0.5 and 3% by weight. In still more preferred embodiments of the invention, a medicament, such as a dermatological formulation, and methods of topical administration of a therapeutically effective amount should comprise Oxaprozin or a closely related compound or a salt thereof in an amount ranging between 1 and 7% by weight, preferably between 1 and 6.5% by weight, such as between 1 and 6% by weight, between 1 and 5.5 % by weight, between 1 and 5% by weight, between 1 and 4.5% by weight, between 1 and 4% by weight, between 1 and 3.5% by weight, such as between 1 and 3% by weight. In still more preferred embodiments of the invention, a medicament, such as a dermatological formulation, and methods of topical administration a therapeutically effective amount should comprise Oxaprozin or a closely related compound or a salt thereof in an amount ranging between 1.5 and 7% by weight, preferably between 1.5 and 6.5% by weight, such as between 1.5 and 6% by weight, 1.5 and 5.5 % by weight, 1.5 and 5% by weight, 1.5 and 4.5% by weight, 1.5 and 4% by weight, 1.5 and 3.5% by weight, such as 1.5 and 3% by weight.

In currently interesting embodiments of the invention, a medicament, such as a dermatological formulation, and methods of topical administration of a therapeutically effective amount should comprise Oxaprozin or a closely related compound in an amount of about 1%, of about 1.5%, of about 2%, of about 2.5%, of about 3%, of about 3.5%, of about 4% by weight, of about 4.5% weight, of about 5% by weight or of about 6% by weight, preferably 2.5%; 3%, 3.5% or 4% by weight.

It should be understood, that in preferred uses and methods of the invention, Oxaprozin or the closely related compound is the sole therapeutically active ingredient.

However, in other uses and methods, Oxaprozin or a closely related compound or a salt thereof is administered together with a dermatological treatment agent. This may be an effective treatment for dermatological diseases and in preferred embodiments the use of the two agents in combination is superior to the results that would be expected based on the use of either agent alone. For example, the combination therapy is effective for lowering the dosages of conventional dermatological agents that are normally prescribed as a mono-therapy. The administration of lower dosages of conventional treatment agents provides a reduction in side effects corresponding to such conventional agents.

Therefore, uses and methods of the invention further comprising the administration of a dermatological treatment agent.

Typical examples on dermatological treatment agents are antihistamines, anti-bacterial agents, anti-fungal agents, anti-pruritus agents, anti-viral agents, agents for combating parasites, steroidal anti-inflammatory agents, non-steroidal anti- inflammatory agents, anaesthetic agents, keratolytic agents, agents for combating free radicals, metal chelating agents, antidandruff agents, anti-acne vulgaris agents, substance P or bradykinin antagonists or NO-synthase inhibitors.

The invention is further described by the examples.

Example 1 describes a typical formulation of a dermatological composition of Oxaprozin in the form of its water-soluble salt (mono-ethanolamine salt) and the formation of the water-soluble salt of Oxaprozin.

Examples 2, 3 and 4 demonstrate the beneficial effect of topical application of Oxaprozin (as a water-soluble salt) in treating pruritus associated with contact dermatitis, atopic dermatitis, insect bite inflammation and psoriasis, respectively.

Example 5 demonstrates the new pharmacological properties of Oxaprozin, which could not be demonstrated for Bufexamac that only inhibits the PDE-IV enzyme.

Example 6 demonstrates the significant effect of Oxaprozin in preventing and treating experimental contact dermatitis in a dose related manner and with stronger effect than observed with betamethasone 17-valerate.

Example 7 demonstrates that Oxaprozin is able to inhibition the formation of ear oedema in an experimental contact dermatitis model, but that no effect could be observed for Bufexamac.

Example 8 demonstrates that Oxaprozin are safe when applied topically to skin and do not cause sensitization reactions, photo toxicity or acute dermal irritation even when applied in high dose.

Example 9 demonstrates that an emulsion of Oxaprozin (Example 1) has good cutaneous tolerance even when applied consecutively in a concentration of 5% for 28 days.

Examples 10 and 11 refer to the clinical assessment of the effect of Oxaprozin in the treatment of hand eczema and contact dermatitis, respectively.

### EXAMPLES

### Example 1

A topical pharmaceutical composition according to the invention was prepared by dissolving 2.5% or 5.0% of the monoethanolamine salt of Oxaprozin in the water phase of the topical emulsion with the following composition (w/w):

| **Hydrophobic phase** | |
|---|---|
| Tween 80™ (Polyoxyethylene sorbitan monooleate) | 1% |
| Span 60™ (emulsifier of the sorbitan ester type) | 2% |
| Medium chain triglycerides (MCT) | 20% |
| Petrolatum, white | 10% |
| Paraffin, light | 10% |
| Cetanol | 4% |

| **Hydrophilic phase** | |
|---|---|
| Oxaprozin monoethanolamine salt | 2.5% |
| Water | 42.5% |
| Xanthan gum | 0.5% |
| Glycerol | 2 % |
| Propylenglycol | 2% |
| Benzylalcohol | 0.5% |

The emulsion was prepared by first heating the lipophilic phase and some of the hydrophilic phase (xanthan gum and water) to 70 degrees Celsius, and mixing them. The remaining hydrophilic phase is heated to 50°C and added subsequently cooling them under agitation.

The monoethanolamine salt was prepared according to the following advantageous method:
10.0 g Oxaprozin was dissolved in 230 ml ethyl acetate under mild heating.
2.3 g of monoethanolamine was dissolved in 30 ml ethyl acetate and added to the Oxaprozin solution under agitation. After a few seconds a significant precipitation could be observed. The solution was allowed to cool for 60 minutes and the salt was collected by filtration and dried.

### Example 2

A 71 year old male subject had been suffering from irritant contact dermatitis for more than 5 years. The dermatitis was usually situated on the legs. The symptoms of the dermatitis was erythema, scaling and significant pruritus.
During the last 5 years the subject had regularly been treated with strong topical steroids with a relatively good therapeutic effect on the erythema, but with no short term effect on the pruritus. During an aggravation of the dermatitis associated with a strong itch, the subject initiated a treatment with the emulsion according to example 1 containing 5.0% of the monoethanolamine salt of Oxaprozin. The subject experienced an immediate and complete alleviation of the pruritus 20 minutes after application of the emulsion of example 1. To maintain this level of efficacy, the subject had to reapply the emulsion three times daily the first day and twice daily during the next two weeks, where the erythema and scaling were gradually reduced. After 14 days of treatment the erythema had completely gone and the treatment was stopped. Two weeks later the erythema had still not reappeared. This indicates a surprisingly good therapeutic effect not only on the pruritus, but also on the underlying disease.

A 3½ year old female had been suffering from atopic dermatitis for at least 2 years. The dermatitis was present in the face and on more than 30% of the body and was characterised by erythema and extensive pruritus. The subject had periodically been treated with hydrocortisone ointment or pimecrolimus cream with some effect on the erythema, but with no short term effect on the pruritus.
During an aggravation of the dermatitis with extensive pruritus, the subject was treated with the emulsion according to example 1 containing 2.5% of the monoethanolamine salt of Oxaprozin. 15 minutes after application of the emulsion, the subject experienced a complete alleviation of the pruritus, which lasted 8 hours. During the next week the treatment was repeated when needed, 1-3 times daily and every time a complete recovery from pruritus was observed. During the week of treatment a significant improvement of erythema was also observed indicating a surprisingly good therapeutic effect not only on the pruritus, but also on the underlying disease.

### Example 3

A 37 year old female, which previously had experienced insect bite inflammation in skin with pruritus and oedema as predominant symptoms, was treated with the emulsion according to example 1 containing 2.5% of the monoethanolamine salt of Oxaprozin following an insect bite by a mosquito. This treatment completely alleviated the pruritus after 20 minutes of application of the emulsion and the oedema disappeared overnight.

Contrarily, treatment of previous mosquito attacks with hydrocortisone ointment did not satisfactorily reduce pruritus and oedema.

### Example 4

A 32 year old male subject had been suffering from plaque psoriasis for more than two years. The disease was apparent on the elbows with erythema, scaling and significant pruritus. During an aggravation of the symptoms the subject initiated a week of twice daily treatment with the emulsion according to claim 1 containing 5.0% of the monoethanolamine salt of Oxaprozin. The subject experienced an immediate and significant relief of pruritus after the first treatment. This level of efficacy was maintained for the entire week of treatment. Furthermore a significant reduction of erythema and scaling was observed. Again this indicated a surprisingly good therapeutic effect not only on the pruritus, but also on the underlying disease.

### Example 5

The anti-inflammatory potential of Oxaprozin was determined by evaluating the inhibitory activity of Oxaprozin against the enzymes *Phosphodiesterase PDEIV, Protein Tyrosine Kinase SYK and Protein Tyrosine kinase ZA70 (ZAP-70).* The enzyme assays were conducted by MSD Pharma Services.

The following concentration of Oxaprozin (as the monoethanolamine salt) resulted in 50% inhibition of the following enzymes (IC₅₀);

| **Enzyme** | **MDS Pharma Service No:** | **IC**_{**50**} |
|---|---|---|
| Phosphodiesterase PDE IV | 154000 | 22 µM |
| Protein Tyrosine Kinase, SYK | 155761 | 28 µM |
| Protein Tyrosine Kinase, ZA70 (ZAP-70) | 155987 | 38 µM |

In comparison, Bufexamac only exhibits inhibitory effect on the PDE-IV enzyme.

### Example 6

Screening for Anti-inflammatory Effect in the Oxazolone induced Mouse Ear Oedema Assay.

The anti-inflammatory activity of Oxaprozin was assessed by topical administration of Oxaprozin to oxazolone induced ear inflammation in mice. This screening method is commonly employed for screening and evaluation of anti-inflammatory drugs, in particular with respect to the inflammation seen in contact dermatitis. Betamethasone-17 valerate was used as the positive control.

Oxaprozin in the form of the monoethanolamine salt was administered topically as a dilution in acetone in a quantity of 250-1000µg/ear. Betamethason was administered topically in quantities of 20µg/ear. Betamethason was applied in the commercial form Celeston valerate ® 0.1%.

### Test Procedure

Day 0
   All groups were immunised with 20 µl oxazolone, 1.6% in ethanol 96% (w/v) on the left and the right ear.
Day 7
   The ear thickness of all mice on both the left and right side was measured with an electronic measuring gauge. All groups were challenged with 20 µl oxazolone (1.6% in ethanol 96% (w/v)) on the left ear and the right ear. Vehicle (acetone) or test article solutions were administered 20 minutes before and 20 minutes after oxazolone challenge.
Day 8
   24 hours after oxazolone challenge the ear thickness of all mice was measured with an electronic measuring gauge.

The groups, doses and animal numbers will be as follows:

| Group | Drug | Dose | Animal numbers |
|---|---|---|---|
| 1 | Vehicle, acetone | - | 1-10 |
| 2 | Monoethanolamine Oxaprozin | 1000µg/ear | 11-20 |
| 3 | Monoethanolamine Oxaprozin | 500µg/ear | 21-30 |
| 4 | Monoethanolamine Oxaprozin | 250µg/ear | 31-40 |
| 5 | Betamethasone 17-valerate | 20µg/ear | 41-50 |

Mean thickness of the ears and standard deviations were calculated. Ear swelling was calculated as the difference between the ear thickness day 7 and day 8. Percent inhibition of the ear swelling was assessed as the difference between the mean ear swelling of group 1 and the mean ear swelling of groups 2 to 5 expressed in percent.

### Statistics

Differences in ear swelling between the vehicle treated group and the other groups were tested for significance employing a non-parametric statistical method of analysis, the Mann-Whitney U test. The required level of significance was p<0.05.

### Results

The oxazolone challenge caused an inflammation in the ears, which was significant in the vehicle treated group after 24 hours since the ears were swollen and bright red. The test articles to some extent prevented the reaction. No adverse reactions to any of the test articles were observed.

### Ear swelling

The various concentrations of the test articles inhibited the ear swelling as shown in the table below:

| Drug | Dose | % inhibition of ear swelling | Mann-Whitney U test |
|---|---|---|---|
| Vehicle, acetone | - | - | - |
| Monoethanolamine Oxaprozin | 1000µg/ear | 95 | P<0.001 |
| Monoethanolamine Oxaprozin | 500µg/ear | 77 | P<0.001 |
| Monoethanolamine Oxaprozin | 250µg/ear | 53 | P<0.001 |
| Betamethasone 17-valerate | 20µg/ear | 66 | P<0.001 |

### Conclusion

The Oxaprozin monoethanolamine salt of the invention displayed a dose dependent and highly significant inhibition of ear swelling. The inhibition observed with the highest dose was significantly stronger than the inhibition obtained with Betamethasone 17-valerate in its clinically used dose level.
The data indicate that the Oxaprozin monoethanolamine salt of the invention has a strong suppressing effect on contact dermatitis.

### Example 7

Comparison of Oxaprozin and Bufexamac in the Oxazolone induced Mouse Ear Oedema Assay.

The anti-inflammatory activity of Oxaprozin in comparison to Bufexamac was assessed using the same test method as described in Example 6. Both test articles were applied in a dose of 500 µg/ear and dissolved in 96% ethanol. Betamethason, as the positive control was administered topically in quantities of 20µg/ear.

### Results:

Oxaprozin showed a statistically significant inhibition of the formation of ear oedema, but Bufexamac did not inhibit the formation of ear oedema at all.

### Example 8

### Acute dermal irritation

A sample of oxaprozin as the monoethanolamine salt was prepared in two concentrations (2.5% and 5% by weight) by dilution with water and tested for acute dermal irritation.

### Procedure:

The sample was applied at a dose of 0.5 mL, on an undamaged skin area of the right flank of each animal. The patch was secured in position with a strip of surgical adhesive tape.
On the left flank an untreated area served as the control.
The skin reactions were evaluated after 1 hour and then after 24, 48 and 72 hours following removal of the patch according to the following grading scale:

### Grading scales:

Erythema (0: No erythema, 1: Slight(barelyperceptible)erythema, 2: Definite erythema, 3: Moderate to severe erythema, 4: Severe erythema (purpie) with formation of eschars (deep lesions) preventing erythema from being grading).
Oedema (0: No oedema, 1: Very slight (barely perceptible) oedema, 2: Slight oedema (contour clearly defined), 3: Moderate oedema (thickness), 4: Severe oedema (thickness greater than 1 mm, surface larger than zone of application)

### Results:

With respect to Oxaprozin (2.5% by weight), no cutaneous reactions (erythema and oedema) were observed irrespective of the examination time.

With respect to Oxaprozin (5% by weight), only a slight erythema on the treated area at the reading time 1 hour was observed. This reaction was totally reversible between the 2nd and the 3rd day of the test.

### Phototoxicity:

Test for phototoxicity is carried out to evaluate the risk of cutaneous reactions on the guinea pig following exposure to ultraviolet radiation.

### Procedure:

Oxaprozin (supplied as its monoethanolamine salt) was diluted in water to produce solutions containing either 2.5% or 5% by weight of the Oxaprozin salt. The solution was applied at a dose of 0.5 mL over the whole right-hand flank of each guinea pig. Thirty minutes after the treatment, the animals were subjected to ultraviolet radiations (UV-B first and then UV-A).

The animals were irradiated with the irradiation source VLX 3W (Biotronic, Vilbert Lourmat) at the Maximal Non Erythemateous Dose (M.N.E.D) 7000 J/cm2 for UV-A and 150mJ/cm2for the UV-B.

### Results:

A macroscopic evaluation of the cutaneous reactions (erythema and oedema) was conducted 24 and 48 hours after irradiation. No macroscopic cutaneous reaction was attributable to photo irritation as compared with the reactions noticed on the reference sites (8-Methoxypsoralen: positive reference and product alone: negative reference). Thus, Oxaprozin is not phototoxic.

### Skin Sensitization

Test for skin sensitization is carried out according to the Magnusson and Kligman method (J. Invest. Dermatol. 1969. 52, 268-276) and in accordance with O.E.C.D. Guideline N° 406 of July 17th, 1992, and the test method B.6 of the 96/54 E.E.C Directive.

### Procedure:

Oxaprozin (supplied as its monoethanolamine salt) was diluted in water to produce solutions containing 2.5% by weight of the Oxaprozin salt.

Albino guinea pigs of Dunkin-Hartley strain were exposed to the test item after an acclimatisation period of at least five days.

The Maximum Non Necrotizing Concentration (M.N.N.C.) was determined by injecting by intradermal route the following concentrations: 2.5%, 1.25%, 0.625%, 0.3125%, 0.1562% and 0.078% diluted in physiological saline solution.

Pre-Maximum Non Irritant Concentration (pre-M.N.I.C.) was determined by application of the test item under an occlusive dressing during 24 hours, at the following concentrations: 2.5%, 1.25%, 0.625%, 0.3125% diluted in physiological saline solution.

Maximum Non Irritant Concentration (M.N.I.C.) was determined by initially establishing an induction phase by intradermal injection with a physiological saline solution and by topical application of distilled water followed by a 18-day rest phase. In the challenge phase where the test item is under occlusive dressing for 24 hours, the test item was applied to the skin of the Albino guinea pigs at the following concentrations: 2.5%, 1.25%, 0.625%, 0.3125% diluted in physiological saline solution.

### Results:

No macroscopic cutaneous reactions attributable to allergy was recorded during the examination following the removal of the occlusive dressing (challenge phase) from the animals of the treated group. No cutaneous intolerance reaction was recorded in animals from the negative control group. Thus, Oxaprozin monoethanolamine salt is found to not causing sensitization reactions.

### Example 9

Cutaneous tolerance of an emulsion (Example 1) containing Oxaprozin (as the monoethanolamine salt) 2.5% and 5% by weight, respectively, was tested by daily application at a dose of 2 ml per animal per day for 28 consecutive days on undamaged skin of rabbits.

Macroscopic cutaneous examinations were performed daily during the 28 days just before the daily application of the emulsion. Skin erythema, oedema, dryness, elasticity and skin fold thickness were assessed.

The results obtained showed slight erythema and oedema after some days of treatment but was totally reversed before the 19th and 10th day, respectively. Dryness was noted too in the beginning of the treatment and there was also observed slight skin fold thickening. The investigator concluded that the emulsion, both in 2.5% and 5% concentration, presented good dermal cutaneous tolerance after repeated application for 28 days.

### Example 10

The efficacy of Oxaprozin or a related compound to treat and prevent hand eczema can be tested in a blinded treatment with the study preparation or vehicle twice daily for 4 weeks in 2 treatment groups with chronic hand dermatitis. Half of the patients will be treated with a cream formulation of Oxaprozin, e.g. Oxaprozin monoethanolamine salt in a concentration of 2.5% and the second half with the cream vehicle. Clinical assessment will be performed on day 1 prior to the first treatment (baseline) and following 1, 2, 3 and 4 weeks of treatment. Additionally the patients will answer a questionnaire for determination of the Dermatology Life Quality Index, a patients global assessment will be performed. The dosage to be applied is approximately 25 mg per day and the total dosage amounts to approximately 700 mg.

The clinical assessment can be done according to the HECSI scoring system that is an objective and accurate assessment of the severity of hand eczema. It incorporates both the extent and the intensity of the disease. Each hand is divided into five areas (fingertips, fingers (except the tips), palms, back of hand and wrists). For each of these areas the intensity of each of the clinical signs erythema, induration/papulation, vesicles, fissuring, scaling and edema is graded on the following four point scale: 0 = no skin changes, 1 = mild disease, 2 = moderate, 3 = severe

For each location (total of both hands) a score from 0 to 4 is given for the extent of clinical symptoms with respect to the percentual affected area:
0=0%, 1= 1-25%, 2=26-50%, 3=51-75%, 4=76-100%

Finally the score given for the extent at each location is multiplied by the sum of the intensity of each clinical feature (Erythema (E), Infiltration/papulation (I), Vesicles (V), Fissures (F), Scaling (S), Edema (O).

### Example 11

The efficacy of Oxaprozin or a related compound to treat and prevent contact dermatitis can be assessed clinically in humans in a randomized, controlled double-blind study using test persons with known nickel allergy and with healthy skin in the test area. According to a standard procedure, at least three test fields of healthy skin located on the back are assigned to each test person, in which fields' allergy is provocated by application of nickel II sulfate vaseline and test medication is applied in order to test efficacy. The test medication can be a cream formulation of Oxaprozin, e.g. Oxaprozin monoethanolamine salt in a concentration of 2.5% or 5% where the daily dose applied to the test field is approximately 15 mg and 40 mg, respectively. As positive control can be used Betamethasone 17- valerate Cream 0.1%, where the daily dose applied is approximately 0.6 mg betamethasone/day. Furthermore, active ingredient-free vehicle is also tested. On day 1, study medication, positive control and vehicle is applied to test fields (= pretreatment) in that approximately 200 µl of each study preparation will be applied to the respective test fields, for example by using special test chambers (Finn Chambers®, Epitest Ltd. Oy, Finland, 18 mm inside in diameter.
On the consecutive day the pre-treated test fields will be treated with two concentrations of nickel II sulfate vaseline to induce an allergic reaction or with vaseline for 1 hours. The treatment with the different concentrations of nickel II sulfate vaseline will be performed in an smaller area in the middle of the defined pre-treated test fields using smaller test chambers, e.g. Finn Chambers®, Epitest Ltd. Oy, Finland, 12 mm inside in diameter. Approximately 30 µl nickel II sulfate vaseline or vaseline will be used. After this induction the efficacy of preventative treatment with the study preparations will be assessed. On study day 4 to 7 the test fields will be treated as described for day 1 with the study preparations once daily to assess the efficacy in the treatment of contact dermatitis. The extent of epidermal barrier impairment measured by TEWL, skin redness measured by chromametry and clinical skin condition evaluated by scoring will be determined. In addition photodocumentation will be performed. Clinical assessment will be performed according to the following score:
0 = no reaction, 1 = erythema, but no induration, 2 = erythema, induration, discrete papules possible, 3 = erythema, induration, papules, vesicle, 4 = erythema, induration, confluencing vesicle.

Transepidermal water loss (evaporimetry) is a widely used non-invasive method for evaluation of skin impairment. The epidermis of healthy intact skin represents a barrier that minimizes external water loss. Any impairment of this barrier results in an increase of permeability to water with a corresponding increase of TEWL. Increased TEWL values are to be expected in the lesional test fields in subjects with allergic reactions induced by the treatment with nickel II sulfate vaseline.

## Claims

1. Use of Oxaprozin or a closely related compound or a pharmaceutically acceptable salt thereof for the preparation of a medicament formulated for topical application to skin for the treatment or prevention of pruritus in skin of a subject.
wherein the closely related compound is defined by the general formula I: 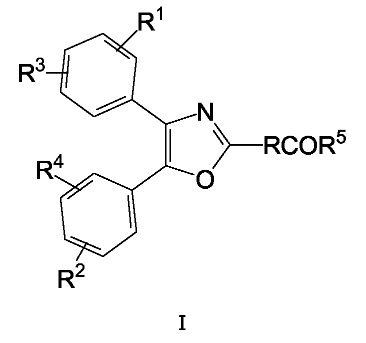 and wherein
R is selected from C₁₋₃-alkyl, C₂₋₃-alkenyl, and C₂₋₃-alkynyl and R is optionally derivatized by substitution of one hydrogen atom with CN, halogen OH, NH₂ and NO₂
R¹ and R² independently designate radicals selected from hydrido, C₁₋₆-alkyl, C₂₋₆₋alkenyl, C₂₋₆-alkynyl, C₁₋₆-alkoxyl, CO, CHO, CO-Me, CO-Et, CN, halogen, OH, OR', NH₂, NHR', NR'R", NO₂, HSO₂ and R⁷-SO₂;
R³ and R⁴ independently designate radicals selected from hydrido, C₁₋₆-alkyl and C₂₋₆₋alkenyl;
R⁵ designate radicals selected from OH, OR⁶, NH₂, NHR', NR'R", SH and SR⁶;
R⁶ designate radicals selected from C₁₋₆-alkyl, C₂₋₆-alkenyl and aryl;
R⁷ designate radicals selected from C₁₋₆-alkyl, aryl, NH₂, NHR' and NR'R";
R' and R" designate the same or different group selected from C₁₋₆-alkyl and C₂₋₆₋alkenyl; and
"aryl" means phenyl or mono-substituted phenyl wherein one hydrogen have been replaced by substituents selected from C₁₋₆-alkyl, C₂₋₆-alkenyl, C₁₋₆-alkoxyl, CO, CHO, CN, halogen, OH, NH₂ and NO₂;
and wherein the oxygen of the oxazole ring optionally is replaced with sulfur (S) to provide a thiazole ring.

2. Use of Oxaprozin or a closely related compound or a pharmaceutically acceptable salt thereof for the preparation of a medicament formulated for systemic administration for the treatment of pruritus in skin of a subject, wherein the closely related compound is defined by the general formula I of claim 1 and wherein R, R¹, R², R³, R⁴, R⁵, R⁶, R⁷, R', R", aryl and bioisoster are as defined in claim 1.

3. The use according to any one of claims 1 or 2, wherein pruritus is associated with or caused by a dermatological disease.

4. The use according to any one of claims 1 or 2, wherein pruritus is caused by or associated with a hypersensitivity reaction in skin.

5. The use according to any one of claims 1 or 2, wherein pruritus is caused by or associated with a type-IV allergy reaction in skin.

6. The use according to any one of claims 1 or 2, wherein pruritus is caused by or associated with a type-I allergy reaction in skin.

7. The use according to any one of claims 1 or 2, wherein pruritus is associated with or caused by dermatological diseases selected from the group consisting of insect bite inflammation, bullous pemphigoid, cutaneous T-cell lymphoma, dermatitis herpetiformis, folliculitis, lichen planus, lichen simplex chronicus, pediculosis, prurigo nodularis, scabies, sunburn and urticaria.

8. The use according to any one of claims 1 or 2, wherein pruritus is associated with asteatotic eczema, senile pruritus, stasis dermatitis, psoriasis, seborrheic dermatitis and seborrhea.

9. The use according to any one of claims 1 or 2, wherein pruritus is associated with systemic medications.

10. The use according to any one of claims 1 or 2, wherein pruritus is associated with exposure to water.

11. The use according to any one of claims 1 or 2, wherein pruritus is associated with a systemic disease selected from the group consisting of diabetes, hyperthyroidism, hypothyroidism, disorders of the parathyroid gland, carcinoid syndrome, hepatic disease, pregnancy, intrahepatic cholestasis, obstructive jaundice, primary biliary cirrhosis, drug induced cholestasis, chronic renal failure, uraemia, polycythaemia vera, iron deficiency, Hodgkin's Disease, Mycosis fungoides, Lymphosarcoma, Chronic leukaemia, Myleomatosis, Paraproteinaemia, Mast cell disease, HIV, T-cell lymphoma, leukaemia, multiple myeloma, Waldenstrom's macroglobinaemia, mycosis fungoides, benign gammopathy, systemic mastocytosis, haematological and lymphoproliferative disorders,carcinomatosis, adenocarcinoma and squamous cell carcinoma of various organ, tumour of brain, multiple sclerosis and brain tumours.

12. Use of Oxaprozin or a closely related compound or a pharmaceutically acceptable salt thereof for the preparation of a medicament formulated for topical administration to skin for the treatment or prevention of one or more of the symptoms selected from the group consisting of pruritus, erythema, oedema and scaling in a subject having a dermatological disease.

13. Use of Oxaprozin or a closely related compound or a pharmaceutically acceptable salt thereof for the preparation of a medicament formulated for systemic administration for the treatment or prevention of one or more of the symptoms selected from the group consisting of pruritus, erythema, oedema and scaling in a subject having a dermatological disease.

14. The use according to any one of claims 12 or 13, wherein the dermatological disease is psoriasis.

15. The use according to any of the preceding claims, wherein the medicament formulated for topical administration to skin comprises an amount of Oxaprozin or a closely related compound or a salt thereof ranging between 0.5% and 10% by weight.

16. The use according to any one of the claims 1 to 14, wherein the medicament comprises an amount of the Oxaprozin or closely related compound or a salt thereof of about 2.5% by weight.

17. The use according to any one of the claims 1 to 14, wherein the medicament comprises an amount of the Oxaprozin or closely related compound or a salt thereof of about 5% by weight.

18. The use according to any of the preceding claims, wherein the Oxaprozin or a closely related compound is provided in the form of a water-soluble salt.

19. The use according to any one of the preceding claims, wherein the closely related compound is selected from the group consisting of
4,5-diphenylthiazol-2-yl- propionic acid, optionally in the form of its ethyl or methyl ester;
4,5-diphenyloxazol-2-yl-acrylic acid;
4,5-diphenyloxazol-2-yl-acetic acid;
4,5-di-(4'-chlorophenyl)-oxazol-2-yl-propionic acid;
4,5-diphenyloxazol-2-yl)-propionamide;
4,5-diphenyloxazol-2-yl)-acrylic acid ethyl ester;
4-(4'-bromophenyl)-5-phenyloxazole-2-yl-propionic acid, optionally in the form of its methyl ester;
4-(4-hydroxyphenyl-5-phenyl-2-oxazole propanoic acid, optionally in the form of its ethyl or methyl ester;
4-(4-fluorophenyl)-5-[4-(methylsulfonyl)phenyl]-2-oxazolepropionic acid, optionally in the form of its methyl ester;
4-(4-fluorophenyl)-5-[4-(methylsulfonyl)-phenyl]-2-oxazoleacetic acid, optionally in the form of its ethyl ester;
4-(4-fluorophenyl)-5-[4-(methylsulfonyl)phenyl]-2-oxazolebutanoic acid, optionally in the form of its methyl ester;
4-(4-fluorophenyl)-5-(4-(methylsulfonyl)phenyl)-2-oxazolepropionic amide;
[4-(4-aminosulfonylphenyl)-5-(3,4-dichlorophenyl)]-2-oxazoleacetic acid, optionally in the form of its ethyl or methyl ester;
[4-(4-aminosulfonylphenyl)-5-(3-chloro-4-fluorophenyl)]-2-oxazoleacetic acid, optionally in the form of its ethyl or methyl ester;
[4-(4-aminosulfonylphenyl)-5-(3-fluoro-4-methoxyphenyl)]-2-oxazoleacetic acid, optionally in the form of its ethyl or methyl ester;
[4-(4-aminosulfonylphenyl)-5-(4-chlorophenyl)]-2-oxazoleacetic acid, optionally in the form of its ethyl or methyl ester;
[4-(4-aminosulfonylphenyl)-5-phenyl]-2-oxazolebutanoic acid, optionally in the form of its ethyl or methyl ester;
[4-(4-aminosulfonylphenyl)-5-phenyl]-2-oxazolepropanoic acid, optionally in the form of its ethyl or methyl ester;
[4-(4-aminosulfonylphenyl-5-(3,4-difluorophenyl)]-2-oxazoleacetic acid, optionally in the form of its ethyl or methyl ester;
[4-(4-methylsulfonylphenyl)-5-phenyl]-2-oxazoleacetic acid, optionally in the form of its ethyl or methyl ester;
[4-(4-methylsulfonylphenyl)-5-phenyl]-2-oxazolebutanoic acid, optionally in the form of its ethyl or methyl ester;
[4-(4-methylsulfonylphenyl)-5-phenyl]-2-oxazolepropanoic acid, optionally in the form of its ethyl or methyl ester;
4-[4-aminosulfonylphenyl)-5-(3-fluoro-4-methoxyphenyl)-2-oxazolyl].alpha.-bromoacetic acid, optionally in the form of its ethyl or methyl ester;
5-(4-nitrophenyl-4-phenyl-2-oxazole-2-yl propionic acid, optionally in the form of its ethyl or methyl ester;
5-(4'-fluorophenyl)-4-phenyloxazole-2-yl-propionic acid, optionally in the form of its methyl ester;
5-(4-hydroxyphenyl-4-phenyl-2-oxazole propanoic acid, optionally in the form of its ethyl or methyl ester;
5-(4-fluorophenyl)-4-[4-(methylsulfonyl) phenyl]-2-oxazolepropionic acid, optionally in the form of its ethyl or methyl ester;
[5-(4-aminosulfonylphenyl)-4-(4-chlorophenyl)]-2-oxazoleacetic acid, optionally in the form of its ethyl or methyl ester;
[5-(4-aminosulfonylphenyl)-4-phenyl]-2-oxazoleacetic acid, optionally in the form of its ethyl or methyl ester;
[5-(4-aminosulfonylphenyl)-4-phenyl]-2-oxazolebutanoic acid, optionally in the form of its ethyl or methyl ester;
[5-(4-aminosulfonylphenyl)-4-phenyl]-2-oxazolepropanoic acid, optionally in the form of its ethyl or methyl ester;
[5-(4-aminosulfonylphenyl)-4-phenyl]-2-oxazolepropionic acid, optionally in the form of its ethyl or methyl ester;
ethyl [4-(4-aminosulfonylphenyl)-5-(3-fluoro-4-methoxyphenyl)]-2-oxazoleacetate;
ethyl [4-(4-aminosulfonylphenyl)-5-phenyl]-2-oxazoleacetate;
ethyl [4-(4-aminosulfonylphenyl)-5-phenyl]-2-oxazolebutanoate;
ethyl [4-(4-aminosulfonylphenyl)-5-phenyl]-2-oxazolepropanoate;
ethyl [4-(4-methylsulfonylphenyl)-5-phenyl]-2-oxazoleacetate;
ethyl [4-(4-methylsulfonylphenyl)-5-phenyl]-2-oxazolebutanoate;
ethyl [4-(4-methylsulfonylphenyl)-5-phenyl]-2-oxazolepropanoate;
ethyl [5-(4-chlorophenyl) - 4-phenylthiazol] 2-yl propionic acid;
ethyl [5-(4-aminosulfonylphenyl)-4-phenyl]-2-oxazoleacetate;
ethyl [5-(4-aminosulfonylphenyl)-4-phenyl]-2-oxazolebutanoate;
ethyl [5-(4-aminosulfonylphenyl)-4-phenyl]-2-oxazolepropanoate;
methyl [4-(4-aminosulfonylphenyl)-5-phenyl]-2-oxazoleacetate;
methyl [4-(4-aminosulfonylphenyl)-5-phenyl]-2-oxazolebutanoate;
methyl [4-(4-aminosulfonylphenyl)-5-phenyl]-2-oxazolepropanoate;
methyl [4-(4-methylsulfonylphenyl)-5-phenyl]-2-oxazoleacetate;
methyl [4-(4-methylsulfonylphenyl)-5-phenyl]-2-oxazolebutanoate;
methyl [4-(4-methylsulfonylphenyl)-5-phenyl]-2-oxazolepropanoate;
methyl [5-(4-aminosulfonylphenyl)-4-phenyl]-2-oxazoleacetate;
methyl [5-(4-aminosulfonylphenyl)-4-phenyl]-2-oxazolebutanoate;
methyl [5-(4-aminosulfonylphenyl)-4-phenyl]-2-oxazolepropanoate.

20. The use according to any one of the preceding claims, wherein the subject is a human, a dog, a cat or a horse.
